Europäisches Patentamt

European Patent Office

Office européen des brevets

(11) Veröffentlichungsnummer: **0 272 265 B1**

(12) **EUROPÄISCHE PATENTSCHRIFT**

(45) Veröffentlichungstag der Patentschrift:
22.05.91 Patentblatt 91/21

(51) Int. Cl.⁵: **A61K 9/20, A61K 9/46**

(21) Anmeldenummer: **86905790.1**

(22) Anmeldetag: **20.09.86**

(86) Internationale Anmeldenummer:
**PCT/EP86/00551**

(87) Internationale Veröffentlichungsnummer:
**WO 87/01936 09.04.87 Gazette 87/08**

(54) ZERFALLSTABLETTE UND VERFAHREN ZU IHRER HERSTELLUNG.

(30) Priorität: 25.09.85 CH 4153/85

(43) Veröffentlichungstag der Anmeldung:
29.06.88 Patentblatt 88/26

(45) Bekanntmachung des Hinweises auf die
Patenterteilung:
22.05.91 Patentblatt 91/21

(84) Benannte Vertragsstaaten:
AT BE CH DE FR GB IT LI NL SE

(56) Entgegenhaltungen:
EP-A- 0 052 076
DE-A- 2 416 903
FR-A- 2 383 659
FR-A- 2 552 308
GB-A- 825 892
GB-A- 974 917

(73) Patentinhaber: Gergely, Gerhard, Dr.
Gartengasse 8
A-1050 Wien (AT)

(72) Erfinder: GERGELY, Thomas
Gartengasse 8
A-1050 Wien (AT)
Erfinder: GERGELY, Irmgard
Gartengasse 8
A-1050 Wien (AT)
Erfinder: GERGELY, Gerhard
Gartengasse 8
A-1050 Wien (AT)

(74) Vertreter: Büchel, Kurt F., Dr.
Bergstrasse 297
FL-9495 Triesen (LI)

## Beschreibung

Die Erfindung betrifft eine wenigstens je einen langsam, bzw. schwer löslichen pharmazeutischen Wirkstoff und ein Sprengmittel enthaltende Tablette, sowie eine besondere Form der Tablette, und schliesslich ein Verfahren zu ihrer Herstellung. Eine solche Tablette ist z.B. aus der EP-A-52,076 oder der GB-C-825,892 bekanntgeworden. Die FR-A-2 383 659 und DE-C-1 068 863 beschreiben die Herstellung von Tabletten aus einer zerkleinerten Schnelze von Kohlenkydraten, spreng mittel und Wirkstoff. Zu festen Körpern komprimierte Presslinge, wie sie in der Pulvertechnologie üblich sind, sollen nämlich in vielen Fällen zwar hart und mechanisch stabil sein, trotzdem aber in Wasser innerhalb kurzer Zeit in die Tabletten-Komponenten zerfallen, um die Wirkstoffe freizugeben und deren Kontakt mit Wasser oder mit Körperflüssigkeit zu ermöglichen.

Zu diesem Zweck werden, seitdem es Komprimate gibt, die verschiedensten Stoffe eingebaut, um den Zerfall solcher Stoffe herbeizuführen. Der erste und klassisch verwendete Stoff war Stärke. Heute gibt es eine Unzahl von Stoffen, die für den Zerfall von Tabletten verwendet werden, wie z.B. fein disperse Kieselsäure, Mikrozellulose, oder insbesondere Polyvinylpyrrolidon, das unzweifelhaft die beste Zerfallswirkung zeigt. Die mechanische Wirksamkeit all dieser Sprengmittel ist jedoch stark abhängig von der Wasserlöslichkeit der Komprimat-Inhaltsstoffe. Paradoxerweise wird die Auswahl eines geeigneten Sprengmittels schwieriger und der Zerfall solcher Komprimate langsamer und unzuverlässiger, je grösser die Wasserlöslichkeit der Inhaltsoder Begleitstoffe ist. Dies ist möglicherweise dadurch bedingt, dass die an der Komprimatoberfläche entstehende, konzentrierte und daher oft hochviskose (z.B. Zucker!) Lösung solcher Stoffe die Kapillaren des Sprengmittels unmittelbar verschliesst und verstopft.

Man führte zwar früher die Wirkungsweise solcher Sprengmittel auf ihre Quellwirkung zurück ; heute ist man allerdings eher der Meinung, dass es an den Sprengmittelteilen angreifende kapillaraktive Kräfte sind, die an gewissen Stellen der Tablette die Bindungskräfte herabsetzen und den Zerfall herbeiführen. Wahrscheinlich wird aber trotzdem zusätzlich zur kapillaraktiven Wirkung eine Volumsvermehrung zum Zerfall der Tablette notwendig sein, sodass es sich um das Zusammenwirken mehrerer Faktoren handelt. Nun wird aber die Kapillaraktivität, die kaum messbar ist, durch sehr viele Einflüsse bei der Herstellung von Präparaten oder beim Pressen der Tabletten negativ beeinflusst. Vor allem sind es höhere Pressdrücke, Granulierungshilfsmittel etc., die die Kapillaren verschliessen und dadurch die Sprengwirkung herabsetzen.

Durch diese und andere Umstände war es bisher kaum möglich, Tabletten, die grössere Mengen wasserlöslicher Kohlehydrate enthalten, zum raschen Zerfall zu bringen. Andererseits besteht aber in der modernen pharmazeutischen Technologie der Wunsch nach solchen Tabletten, die sowohl oral angenehm eingenommen werden können, als auch in Wasser rasch zerfallen, wobei ihre Bestandteile sich entweder auflösen oder zumindest dispergiert bleiben und ein Getränk ergeben. Diesen Wunsch konnten aber die Tabletten nach den beiden eingangs erwähnten Schriften vom Stande der Technik nicht entsprechen. Es war nämlich bisher kaum möglich, ein Zwischenglied zwischen der normalen Tablette oder Kapsel, die entweder geschluckt wird oder in Wasser lediglich zerfällt, und einer sich auflösenden, ein wohlschmeckendes Getränk ergebenden Brausetablette als Darreichungsform zu finden. Ziel der vorliegenden Erfindung ist es daher, eine neue Tablette zu schaffen, die sowohl als solche eingenommen, gekaut oder gelutscht werden kann, als auch in Wasser innerhalb von 30 bis 60 Sekunden zerfällt und eine wohlschmeckende Suspension oder Lösung ergibt. Weiteres Ziel der Erfindung ist es, die Tablette frei von Glucose oder Saccharose zu machen und gegebenenfalls auf die Verwendung von brausenden Zusätzen verzichten zu können.

Man teilt in der Regel alle Stoffe in leicht- oder schwerlösliche ein, was sprachlich deshalb nicht exakt ist, weil man eigentlich "viel" und "wenig" lösliche damit meint. Es macht nämlich einen grossen Unterschied aus, ob ein Stoff, von dem verhältnismässig viel in z.B. einem Liter Wasser gelöst werden kann, sich in diesem Wasser schnell ("leicht") oder langsam ("schwer") löst. Diesen Unterschied, dem bisher offensichtlich zu wenig Bedeutung Beigemessen wurde, nutzt die Erfindung aus.

Dabei ist aber sogar noch eine weitere Stufe zu beachten : Gewöhnliche Saccharosekörner werden z.B. an der Oberfläche schnell angelöst, wobei sich eine konzentrierte Zuckerlösung ergibt, die das weitere Auflösen der Körner nur mehr langsam zulässt, wenn sie nicht z.B. durch Umrühren verteilt wird. Hat man hingegen einen Stoff wie z.B. geschmolzenes Mannitol (siehe unten), der an der Oberfläche nur langsamer aufgelöst wird, entsteht keine so konzentrierte Lösung ; die gelösten Moleküle können rascher wegdiffundieren und das "Durchlösen" des Kornes ist dann unter Umständen rascher als beim Rohrzucker.

Ueberlegt man sich nämlich den Vorgang des Tablettenzerfalls genauer, dann spielt offensihtlich der Zeitablauf der Vorgänge eine grosse Rolle. Gleichgültig, ob es sich nun um eine Quellwirkung oder um eine kapillaraktive Wirkung handelt, muss sicherlich zuerst der Zerfalls-Hilfsstoff benetzt und vom Wasser durchdrungen werden, bevor die Begleitstoffe in Lösung zu gehen beginnen. Würden nämlich die Begleitstoffe schneller oder zumindest zugleich und im selben Ausmass in Lösung gehen, dann wird die Volumsverminderung, die durch das Auflösen der Begleitstoffe eintritt, den Zerfall der Tablette verhindern, bzw. die Wirksamkeit der Sprengmittel herabsetzen. Dazu kommt noch, dass wasserlösliche Stoffe beim Lösungsvorgang eher Bindemitteleigenschaften zeigen und auch bei kapillaraktiven Stoffen, wie erwähnt, den Zerfall behindern.

Es war daher bisher nur sehr schwer möglich, z.B. Kohlehydrate in rasch zerfallende Tablettenformen eizubringen, da dort das Auflösen der leicht wasserlöslichen Kohlehydrate fast alle Sprengmittel an ihrer Wirksamkeit hindert. Eine solche Tablette wird nun aber durch die Massnahmen des Kennzeichens des Anspruchs 1 geschaffen.

Es kann sich bei den Füllstoffkörnern um besonders grobe Kristalle (z.B. 0,2 bis 0,6 mm Korngrösse) oder um andere Kristallmodifikationen handeln, die langsamer in Lösung gehen, als feine Kristalle, bzw. als das üblicherweise vorliegende Pulver. Es ist auch möglich, die Körner leichtlöslicher Stoffe – z.B. mit Hilfe an sich bekannter Vakuumtechnologie – mit einem hauchdünnen Ueberzug eines pharmazeutisch unbedenklichen, langsam löslichen Materials, z.B. mit einem Kolloid oder Pseudokolloid oder dergleichen zu beschichten. Wesentlich ist immer, dass das Wasser zuerst an die in der gepressten Tablettenmischung vorhandenen Sprengmittelteilchen gelangt und diese zur Quellung und damit zum Sprengen der Tablette bringt, bevor die gegebenenfalls schnell löslichen Wirk- oder Füllstoffe von dem Wasser gelöst werden; die dann an den Kornoberflächen entstehende hochkonzentrierte Lösung könnte nämlich nicht mehr in die Sprengmittelkapillaren eindringen.

Es ist aber auch Sorge dafür zu tragen, dass die gegebenenfalls rasch quellenden Kolloide nicht vielleicht die aus der Kornoberfläche ragenden Sprengmittelteile mit einer undurchdringlichen Gallerte überziehen, bevor das Sprengmittel zur Wirkung kommen konnte.

Vorteilhafte Weiterbildungen der erfindungsgemässen Tablette sind in den Ansprüchen 2 bis 4, ein Verfahren zur Herstellung der erfindungsgemässen Tablette in Anspruch 5 beschrieben.

Die gegenständliche Erfindung sieht vor, dass der schnell lösliche Füllstoff durch Aufschmelzen, Abkühlen und Mahlen langsam löslich wird. Dabei ergeben sich zwei Vorteile :

– zum einen erhält die Oberfläche häufig eine unregelmässige, teilamorphe oder mikrokristalline Struktur (im Gegensatz zu den glatten, harten Kristallflächen), wodurch sich die Mischungen viel besser zu Tabletten verpressen lassen, oft sogar ohne Zusatz eines Bindemittels ("direct pressing material") ; übliches Kristallgranulat lässt sich allein so gut wie nicht verpressen, sondern erfordert eigentlich immer ein Bindemittel. Der Bindemittelzusatz aber bedeutet in der Herstellung oft einen zusätzlichen Arbeitsgang ;

– zum anderen kann das Sprengmittel gleich in die Schmelze eingetragen werden, so dass nach dem Abkühlen und Mahlen an den Kornbruchflächen freie Sprengmittelreste liegen, die ins Korninnere ragen. Die beim Tablettenpressen gebildete Kontaktfläche zwischen den einzelnen Körnern ist von den an der Oberfläche freiliegenden Sprengmittelteilchen unterbrochen. Tritt nun Wasser zwischen die Körner ein, quellen die Sprengmittelteilchen sofort und vereinzeln die Körner ; diese werden dadurch rascher löslich, dass die ins jeweilige Korninnere ragenden Sprengmittelteilchen quellen und die Körner in kleinere Teilchen zersprengen. Dabei werden gleichzeitig die Wirkstoffe freigesetzt und gehen in Lösung oder Suspension.

Die Körner sollen daher in der Tablette nicht zu dicht gepackt sein, damit das Wasser in die Poren zwischen die Körner eindringen kann. Insbesondere wenn es sich ohne Bindemittelzusatz um eine rein elektrisch-mechanische Bindung zwischen den Körnern handelt, ist diese leichter aufzuheben als eine Bindung aus Kolloiden.

Ein besonders einprägsames Beispiel dafür ist Mannitol. Während sich pulverisiertes Mannitol, wie es im Handel erhältlich ist, sehr leicht und schnell in Wasser löst, kann man Mannitol langsam löslich machen, indem man es aufschmilzt, die Schmelze rasch abkühlen lässt und sie dann in speziellen Mühlen, z.B. zu Korngrössen zwischen 0,2 und 0,6 mm, vermahlt. Es liegt auf der Hand, dass normales Mannitol, das eine Korngrösse in der Grössenordnung von 10 micron zeigt, schon aufgrund der grösseren Oberfläche rascher löslich sein wird als geschmolzenes Mannitol, das durch seine kompakte physikalische Struktur und die geringere Oberfläche der geschmolzenen Teilchen zwar ebenso leicht, aber eben langsamer wasserlöslich wird.

Wichtig ist natürlich auch, dass beim Verpressen von bindemittelfreien Körnern aus geschmolzenem Mannitol zu einer Tablette kein Magnesiumstearat als Gleitmittel zugesetzt werden braucht, das die Tablette an der Oberfläche hydrophobieren und ihren Zerfall beim späteren Kontakt mit Wasser ausserordentlich verlangsamen würde.

Es ist nun in sehr eleganter Weise möglich, die Auflösungsgeschwindigkeit eines derartigen Stoffes wie Mannitol noch weiter herabzusetzen, und zwar (paradoxerweise) durch Hinzufügen eines Sprengmittels. Dazu wird geschmolzenes Mannitol mit einem Zusatz von beispielsweise 10-20% hochgereinigter Mikrozellulose versehen. Diese Mikrozellulose wird mit einem hochwirksamen Rührwerk in das geschmolzene Mannitol eingetragen, das Mannitol hernach wiederum abgeschreckt und vermahlen. Die Mikrozellulosepartikel werden an das bzw. in das Mannitol an-(ein-)geklammert, wodurch das Mannitol in der Schmelze noch kompakter und dadurch noch langsamer löslich wird. Presst man nun solche Partikel zu einer Tablette unter möglichem Zusatz eines weiteren Sprengmittels, dann wird unter Einwirkung von Wasser das an der Grenzfläche befindliche Zerfallsmittel rascher quellen als die An- oder Auflösung des durch das Schmelzen kompakter und daher ziemlich langsam löslich gewordenen Mannitols geschieht. Dadurch beginnt die Tablette sofort zu zerfallen, wobei sich die Mannitol-Partikel zunächst in Wasser verteilen und erst nachher auflösen. In eine solche Tablette können eine Vielzahl von Wirkstoffen eingearbeitet werden, die sich dann ebenfalls entweder auflösen oder in Wasser suspendiert werden ; man erreicht so auch bei leicht wasserlöslichen Füllstoffen Zerfallsgeschwindigkeiten von 30 bis 60 Sekunden, trotzdem die Presslinge auf beispielsweise 10 bis 15 kp Härte überaus hart verpresst sein können.

Man hat zwar schon bisher Wirkstoffe schmelzversprüht (z.B. DE-A-2416,903), um sie besser tablettierbar zu machen. Nach der FR-A-2383659 werden verschiedene Polysaccharide in Mischung mit einem sauerstoffempfindlichen Wirkstoff durch Erhitzen auf 80 Grad C komprimiert, wobei aber in Wirklichkeit die Polysaccharide nicht schmelzen ; man zerkleinert dann wieder und verbessert damit die Lagerfähigkeit des Wirkstoffes. Man hat dabei auch verschiedene Hilfsstoffe, wie z.B. Aromen, künstliche Süssstoffe, Verdickungs- oder Bindemittel, Antikleb- oder Gleitmittel, etc. zugesetzt, nicht aber **langsam** lösliche **Füllstoffe**, wie dies die erfindungsgemässen Vorteile bringt. Erfindungsgemäss hat sich allerdings gezeigt, dass bis zu maximal einem Drittel, vorzugsweise allerdings maximal 20% der Gesamtmenge der Tablette auch aus den erwähnten Hilfsstoffen bestehen kann, von denen einige verhältnismässig schnell wasserlöslich sind.

Das neue Zerfallsprinzip hat eine wesentliche Bedeutung im Aufbau neuer Komprimat-Systeme, die eine zweifache Anwendung erlauben. Man kann nun solche Komprimate, wenn gewünscht, in einer verhältnismässig sehr geringen Menge Wasser zum Zerfall bringen, wobei sich innerhalb von 30 bis 60 Sekunden eine Suspension der Tabletteninhaltsstoffe bildet ; man kann sie aber auch schlucken, kauen oder lutschen.

Der Effekt ist auch deshalb bemerkenswert, weil eine derartige Tablette im Mund ein ganz anderes Verhalten zeigt. Hier kommt es nämlich durch die geringe Feuchtigkeitsmenge des Speichels nicht zum Zerfall, sondern die Tablette benimmt sich wie eine normale Kau- oder Lutschtablette, die angenehm einzunehmen ist.

Der Vorzug des Systems liegt darin, dass es erstens in der Anwendung und Herstellung billig ist und vor allem geschmacklich jede Variationsbreite erlaubt. Die neuerdings viel verwendeten quervernetzten Polyvinylpyrrolidone, die die Bildung ähnlicher Systeme erlauben, haben den Nachteil eines hohen Preises und eines sandigen Geschmackes, der bei der Einnahme sehr störend wirkt. Ausserdem versagen solche quervernetzte Polyvinylpyrrolidone in Anwesenheit leicht (schnell) löslicher Substanzen.

Die Variationsbreite des soeben geschilderten Verfahrens ist enorm. Viele Kohlehydrate (wie übrigens auch sehr viele Wirkstoffe) lassen sich ohne Zersetzung aufschmelzen und dabei mit Sprengmitteln versetzen. Man lässt die Schmelze dann erstarren und mahlt auf die gewünschte, für die Verpressung zu den betreffenden Tabletten optimale Korngrösse. Im allgemeinen genügen wenige Vorversuche, um für ein gewünschtes System ein passendes Modell zu finden.

Es liegt auf der Hand, dass vermittels dieses Systems eine grosse Reihe von Zerfallstabletten aufgebaut werden kann, weil nahezu sämtliche Sprengmittel, die bisher zum Zerfall von Tabletten verwendet werden, im Falle ihrer Temperaturverträglichkeit in die Schmelze von Kohlehydraten eingearbeitet werden können. Ausserdem sind solche Systeme relativ unempfindlich im Auflöseverhalten gegen Süss- und Aromastoffe und können daher zur Herstellung wohlschmeckender Produkte dienen.

Hervorzuheben ist noch, dass solche Systeme aufgrund der geringeren Oberfläche und der höheren Kompaktheit (Dichtigkeit) der Schmelzgranulate wesentlich weniger feuchtigkeitsempfindlich sind als normale Zerfallstabletten. Der Grund liegt natürlich darin, dass auch normale Luftfeuchtigkeit auf die geringere Oberfläche der Schmelzkomprimate weniger einwirken kann als auf komprimierte Teilchen kleinerer Korngrösse.

Die erfindungsgemässen Füllstoffe mit Sprengmittel können in ganz zweckmässiger Weise auch für die Herstellung der nicht brausenden Schicht von Mehrschicht-Brausetabletten Verwendung finden. In der modernen Heilkunde ist man nämlich in zunehmendem Masse bestrebt, pharmazeutische Wirkstoffe in Brausetabletten unterzubringen, weil die orale Einnahme von Tabletten, insbesondere wenn es sich um hohe Dosen handelt, bei vielen Patienten auf Schwierigkeiten stösst, während Brauselimonaden gern getrunken werden ; weiters kann auf diese Weise die an sich erwünschte zusätzliche Einnahme einer ausreichenden Menge Wassers sichergestellt werden.

Nun hat es sich leider gezeigt, dass verschiedene Wirkstoffe nebeneinander und/oder in Brausemischungen gar nicht oder nur beschränkt stabil sind. Man hat daher auch schon vorgeschlagen, eine Mehrschichttablette vorzusehen, bei der die Brausemischung und/oder ein Wirkstoff in einer, der oder die anderen Wirkstoff(e) in einer anderen Schicht angeordnet sind. Diese Lösung scheidet aber in vielen Fällen aus, weil die in der anderen Schicht angeordneten Wirkstoffe, insbesondere in der gepressten Form, nicht so schnell wasserlöslich sind, wie die Brausemischung sich auflöst.

Bildet man nun die brausemittelfreie Schicht in erfindungsgemässer Weise aus, dann dringt in derselben Zeit, in der die Brauseschicht sich auflöst, das Wasser durch die Dochtwirkung des Sprengmittels auch in die keine Brausemischung enthaltende Tablettenschicht ein und zersprengt diese.

Will man z.B. Acetylsalicylsäure in einer natriumarmen Brausetablette unterbringen, ist dies deswegen schwierig, weil sie unter Einwirkung des stärker alkalisch reagierenden Kalziumcarbonates stärker verseift wird. Man kann daher z.B. die Brausemischung in der einen Schicht, die Acetylsalicylsäure in der anderen, brausemittelfreien Schicht unterbringen, was aber nur dann sinnvoll ist, wenn diese letztere Schicht erfindungsgemäss aufgebaut ist. Ansonsten bestünde die Gefahr, dass die brausemittelfreie Schicht nicht so schnell zerfällt wie die brausemittelhältige Schicht sich auflöst und daher als Einzeltablette übrig bleibt, während sich die erste Schichte beim Brausen von dieser zweiten Schichte löst. Dazu kommt noch, dass die Acetylsalicylsäure Kohlensäure benötigt, um sich aufzulösen, da sie von sich aus hydrophob und dadurch relativ schwer löslich ist.

Besonders zweckmässig ist es in diesem Fall, wenn eine brausemittelfreie Schicht auf beiden Seiten von je einer eine Brausemischung enthaltenden Schicht bedeckt ist, weil dann auf jeden Fall auch auf der Unterseite der brausemittelfreien Schicht Kohlensäure gebildet wird und ein zusätzlicher "Rühreffekt" für das Zerfallen und Auflösen der brausemittelfreien, sprengmittelhältigen Mittelschicht besteht. Bei nur einseitiger Brausemittelschicht "schwimmt" diese nämlich immer auf der Oberseite und der Rühreffekt für die darunter hängende brausemittelfreie Schicht unterbleibt.

Beispiel 1 :

90 Teile Mannitol werden bei 180 Grad im Oelbad geschmolzen ; mittels eines hochwirksamen Rührwerks werden 10 Teile Mikrozellulose in der Schmelze suspendiert. Die Schmelze wird auf gekühlte Tassen gegossen, wo sie alsbald erstarrt. Die Zerkleinerung der Masse erfolgt durch eine Messermühle. Vorzugsweise werden die Partikel zwischen 0,2 und 0,6 mm verwendet. 400 mg dieser Mannitol-Zellulose-Schmelze werden nun mit 200 mg Erythromycinsuccinat und weiteren 50 mg Mikrozellulose versetzt und ohne weiteren Bindemittelzusatz tablettiert. Die Tablette gibt mit einem Stempel von 12 mm Durchmesser eine Härte von etwa 10 kg und zerfällt in Wasser innerhalb von 15-30 Sekunden. Die Zugabe von Aromastoffen und Süssstoffen verändert die Zerfallsgeschwindigkeit und die Eigenschaften der Tablette nicht.

Beispiel 2 :

200 mg Penicillin V werden mit 300 mg des Mannitol-Zellulose-Produktes aus Beispiel 1, 50 mg Stärke und 30 mg Fumarsäure, sowie mit üblichen Aroma- und Süssstoffen verpresst. Die Härte der Tablette beträgt 10 kg, die Zerfallszeit 20-30 Sekunden.

Beispiel 3

Besonders vorteilhaft kann auch Stärke in die Mannitol-Schmelze eingebaut werden. 92 Teile Mannitol werden bei 180 Grad geschmolzen ; man bringt nun langsam 8 Teile Kartoffelstärke ein und suspendiert sie durch Rühren. Hier ergibt sich der weitere Vorteil, dass das in der Stärke enthaltene Wasser verdampft, wodurch die Stärke trocknet und ausserordentlich zerfallswirksam wird. Die Masse wird wiederum gekühlt und die erstarrte Schmelze zu Partikeln zwischen 0,2 und 0,5 mm gemahlen.

Mit diesem System lassen sich vornehmlich hydrophobe Produkte verarbeiten, wie beispielsweise Vitamin-E-Adsorbate. Zum Beispiel werden 400 mg 50%iges Vitamin-E-Adsorbat an Gelatine und 1000 mg des Mannitol-Stärkeproduktes mit Geschmacks- und Süssstoffen zu einer Tablette verpresst. Die Härte der Tablette beträgt 8 kg, die Zerfallszeit 20 Sekunden.

Beispiel 4 :

90 Teile Zitronensäure, wasserfrei, werden bei 150 Grad geschmolzen ; man bringt in die Schmelze 10 Teile feindisperses Siliziumoxyd ein und schreckt ab. Das erhaltene Produkt wird zu einer Korngrösse von 0,2 bis 0,5 mm vermahlen.

Aus diesem Produkt lassen sich Zerfallstabletten aufbauen, die beispielsweise aus 3 Teilen der Mannitol-Zellulose-Schmelze aus Beispiel 1, 1 Teil der oben erwähnten Zitronensäure-Siliziumoxyd-Schmelze und 1 Teil Vitamin C, sowie zusätzlich 0,2 Teilen Microzellulose bestehen.

Die Mischung wird zu Tabletten verpresst, die innerhalb von 30 bis 45 Sekunden in ihre Bestandteile zerfallen. Würde die leicht (schnell !) lösliche Zitronensäure in nicht-geschmolzener Form eingesetzt, dann würde der Tablettenzerfall ausserordentlich verlangsamt.

Beispiel 5 :

Auch Adipinsäure kann ähnlich wie Zitronensäure behandelt werden, wobei die Schmelze sowohl mit Zellulose als auch mit Aerosil im Verhältnis von 10 bis 20% versetzt werden kann. Wird eine mikrozellulosehältige Schmelze mit diesen Bestandteilen abgeschreckt und zu einer Korngrösse von 0,2 bis 0,5% vermahlen, dann eignet sie sich ebenso wie Zitronensäure zum Ansäuern von leicht zerfallenden Instant-Tabletten.

**Beispiel 7 :**

60 Teile Xylit werden bei 160 Grad mit 40 Teilen Stärke verrührt und abgeschreckt. Das erhaltene Gut wird zu Teilchen von 0,2 bis 0,5 mm vermahlen und ergibt mit einem weiteren Zusatz von 10 bis 20% Stärke und üblichen Wirk-, Aroma- und/oder Süssstoffen harte Tabletten, die in einem Zeitraum von 30-40 Sekunden zerfallen, soferne die Wirk-, Aroma-oder Süssstoffe nicht schnell löslich sind.

**Ansprüche**

1. Wenigstens je einen im Wasser langsam, bzw. schwerlöslichen pharmazeutischen Wirkstoff und ein Sprengmittel enthaltende Tablette, dadurch gekennzeichnet, dass das Sprengmittel in einer Schmelze dispergiert ist, die aus wenigstens einer der Substanzen Mannitol, Sorbitol und Xylitol besteht, welche Schmelze in zerkleinerten Form vorliegt, wobei maximal ein Drittel der Tablette aus im Wasser schnelllöslichen Hilfsstoffen besteht.

2. Tablette nach Anspruch 1, dadurch gekennzeichnet, dass in der zerkleinerten Schmelze auch wenigstens ein bei der Schmelz- temperatur und -dauer stabiler Wirkstoff dispergiert ist.

3. Tablette nach Anspruch 1 oder 2, dadurch gekennzeichnet, dass sie eine zerkleinerte Schmelze in Mischung mit wenigstens einer anderen zerkleinerten Schmelze und/oder wenigstens einem Sprengmittel enthält.

4. Mehrschichttablette, enthaltend wenigstens eine Schicht in Form einer Tablette nach einem der vorhergehenden Ansprüche, dadurch gekennzeichnet, dass sie wenigstens auf einer Seite von einer eine Brausemischung enthaltenden Schicht abgedeckt ist.

5. Verfahren zur Herstellung einer Tablette nach einem der Ansprüche 1 bis 3, dadurch gekennzeichnet, dass die die Matrix bildenden Substanzen – und gegebenenfalls der wenigstens eine Wirkstoff – bei einer Temperatur unterhalb der Zersetzungs- temperatur der Bestandteile aufgeschmolzen werden, wobei vor, während oder nach dem Aufschmelzvorgang wenigstens ein Sprengmittel und/oder andere Wirk- und/oder Füllstoffe unter Rühren verteilt, bzw. suspendiert werden, und dass die Schmelze schliesslich abgekühlt, insbesondere abgeschreckt und bei einer Temperatur unterhalb ihres Erweichungspunktes auf die gewünschte Korngrösse zerkleinert wird.

**Claims**

1. Tablet containing at least one pharmaceutical active compound which is slowly or sparingly soluble in water and at least one disintegrating agent, characterized in that the disintegrating agent is dispersed in a melt which consists of at least one of the substances mannitol, sorbitol and xylitol, which melt is present in comminuted form, not more than one third of the tablet consisting of auxiliaries which are rapidly soluble in water.

2. Tablet according to Claim 1, characterized in that at least one active compound which is stable at the melting point and for the melting time is also dispersed in the comminuted melt.

3. Tablet according to Claim 1 or 2, characterized in that it contains a comminuted melt mixed with at least one other comminuted melt and/or at least one disintegrating agent.

4. Multilayer tablet containing at least one layer in the form of a tablet according to one of the preceding Claims, characterized in that it is covered on at least one side by a layer containing an effervescent mixture.

5. A process for the preparation of a tablet according to any of Claims 1 to 3, characterized in that the matrix-forming substances – and optionally the one or more active compounds – are melted at a temperature below the decomposition temperature of the constituents, at least one disintegrating agent and/or other active compounds and/or fillers being distributed or suspended, while stirring, before, during or after the melting process, and that the melt is finally cooled, in particular quenched, and is comminuted to the desired particle size at a temperature below its softening point.

**Revendications**

1. Comprimé comprenant au moins un agent actif pharmaceutique se dissolvant lentement ou difficilement dans l'eau et au moins un agent explosif, caractérisé en ce que l'agent explosif est dispersé dans une masse fondue qui est composée au moins de l'une des substances : Mannitol, Sorbitol et Xylitol, laquelle masse fondue est présente sous forme broyée, au maximum un tiers du comprimé étant composé de matières auxiliaires rapidement solubles dans l'eau.

2. Comprimé selon la revendication 1, caractérisé en ce qu'au moins un agent actif stable dans les conditions de température et de durée de la masse fondue est dispersé également dans la masse fondue broyée.

3. Comprimé selon la revendication 1 ou la revendication 2, caractérisé en ce qu'il contient une masse fondue broyée en mélange avec au moins une autre masse fondue broyée et/ou au moins un agent explosif.

4. Comprimé multi-couches comprenant au moins une couche sous forme de comprimé selon l'une des revendications précédentes, caractérisé en ce qu'il est recouvert, au moins sur un côté, d'une couche contenant un mélange effervescent.

5. Procédé pour la fabrication d'un comprimé selon l'une des revendications 1 à 3, caractérisé en ce que les substances qui forment la masse fondue – et éventuellement au moins cette matière active unique – sont fondues à une température inférieure à la température de décomposition des composants, au moins un agent explosif et/ou d'autres agents actifs et/ou de remplissage étant, avant, pendant ou après la fusion, versés ou mis en suspension sous agitation; et en ce que la masse fondue est finalement refroidie, en particulier trempée, et broyée à la grosseur de grains désirée à une température inférieure à son point de ramollissement.